# EUROPÄISCHE PATENTSCHRIFT

(11) **EP 2 244 652 B1**
(45) Veröffentlichungstag und Bekanntmachung des Hinweises auf die Patenterteilung: **08.02.2017**
(21) Anmeldenummer: 08836292.6
(22) Anmeldetag: 24.09.2008
(51) Int. Cl.: A61B 90/98, A61B 5/00, A61B 90/00, A61B 34/20

(54) **GEWEBEMARKIERUNG**
TISSUE MARKER
MARQUEUR DE TISSU

(30) Priorität: 26.09.2007 DE 102007046186
(43) Veröffentlichungstag der Anmeldung: 03.11.2010
(73) Patentinhaber: amedo smart tracking solutions GmbH, 44799 Bochum (DE)
(72) Erfinder: TRÖSKEN, Volker, 58456 Witten (DE); HASENAU, Laszlo, 44789 Bochum (DE)
(74) Vertreter: Isfort, Olaf
(86) Internationale Anmeldenummer: PCT/EP2008/008071
(87) Internationale Veröffentlichungsnummer: WO 2009/043512

(56) Entgegenhaltungen:
- EP-A- 1 321 097
- WO-A-2007/087447
- WO-A-2007/117478
- WO-A1-01/00101
- WO-A1-2005/104976
- DE-A1-102006 029 122
- US-A- 5 325 873
- US-A1- 2002 083 951
- US-A1- 2003 052 785
- US-A1- 2005 101 946
- US-A1- 2005 195 084
- US-A1- 2007 106 152
- US-A1- 2007 135 711
- US-B1- 7 026 941

## Beschreibung

Die Erfindung betrifft eine Gewebemarkierung zur Markierung einer Läsion im Körpergewebe.

In der Medizin werden häufig operative Eingriffe zur Entnahme von Gewebeproben aus dem Körpergewebe durchgeführt, insbesondere bei der Diagnose und Behandlung von Patienten mit Tumoren, prämalignen Zuständen oder anderen lokal begrenzten Erkrankungen. Typischerweise wird, wenn ein Arzt mittels der bekannten Techniken (Palpation, Röntgen, MRI, Ultraschall usw.) feststellt, dass ein Verdacht auf eine Erkrankung besteht, eine Biopsie durchgeführt, um zu bestimmen, ob die Zellen im Bereich der aufgefundenen Läsion krebsartig sind. Die Biopsie kann eine offene oder eine perkutan durchgeführte Biopsie sein. Bei einer offenen Biopsie wird Gewebemasse im Bereich der Läsion vollständig oder teilweise entfernt. Bei der perkutanen Biopsie, die üblicherweise mit einem nadelförmigen Instrument durchgeführt wird, werden kleine Gewebemengen, gegebenenfalls nur einzelne Zellen, für eine pathologische Untersuchung entfernt. In allen Fällen kommt es für eine präzise Durchführung des operativen Eingriffs darauf an, den Ort der Läsion, das heißt die Position des Zentrums der Läsion, sowie auch die Ränder der Läsion möglichst genau lokalisieren zu können. Die genaue Erkennung der Ränder einer Läsion ist wichtig, um zu gewährleisten, dass bei einem entsprechenden Eingriff die gesamte Läsion entfernt wird.

Aus dem Stand der Technik sind verschiedene Techniken zum Markieren und Lokalisieren von Läsionen im Körpergewebe bekannt.

In der Praxis kommen häufig Drahtführungen zum Einsatz, wie sie in der US 5,221,269 beschrieben sind. Solche Drahtführungen werden insbesondere zum Lokalisieren von Läsionen in der Brust eingesetzt. Die Drahtführung umfasst einen Führungsdraht, der an seinem distalen Ende eine helixförmige Spulenkonfiguration aufweist. Der Führungsdraht wird mittels einer hohlen Nadel in die Brust eingeführt und unter Überwachung durch ein Bildgebungssystem zu der Läsionsstelle geführt. Dort wird dann die helixförmige Spule am distalen Ende des Führungsdrahtes im Körpergewebe verankert. Schließlich wird die Hohlnadel von dem Führungsdraht entfernt, der an der Position im Gewebe im Bereich der Läsion verankert bleibt. Der Draht dient dazu, einen Arzt während eines nachfolgenden operativen Eingriffes zu der Läsionsstelle zu führen. Der Führungsdraht verbleibt im Gewebe und wird erst entfernt, nachdem die Behandlung abgeschlossen ist.

Des Weiteren sind so genannte aktive Gewebemarkierungen aus dem Stand der Technik bekannt. Eine solche Gewebemarkierung ist zum Beispiel in der US 7,135,978 B2 beschrieben. Die Gewebemarkierung besteht aus einer Spule, die Bestandteil eines Hochfrequenz-Resonanzkreises ist. Die Spule wird zur Markierung einer Läsion im Körpergewebe an der entsprechenden Stelle im Körper implantiert. Die Gewebemarkierung ist anhand des von dem Hochfrequenz-Resonanzkreis auf eine entsprechende Anregung hin abgestrahlten elektromagnetischen Feldes drahtlos lokalisierbar. Auch diese Art der Gewebemarkierung hat den Nachteil, dass sie bis zum Abschluss der jeweiligen Therapie im Körpergewebe verbleibt und danach durch einen separaten Eingriff entfernt werden muss. Hierdurch wird der Patient belastet.

In WO2007/087447 wird ein System mit einer Gewebemarkierung nach dem Oberbegriff des unten stehenden Anspruchs 1 offenbart.

Vor diesem Hintergrund ist es Aufgabe der Erfindung, eine verbesserte Gewebemarkierung bereitzustellen. Insbesondere soll eine Gewebemarkierung geschaffen werden, die eine zuverlässige Lokalisierung einer Läsion im Körpergewebe ermöglicht, wobei der Patient durch die Gewebemarkierung möglichst wenig belastet werden soll.

Der Gegenstand der Erfindung wird durch die unabhängigen Ansprüche 1 und 13 definiert. Bevorzugte Ausführungsformen sind in den Unteransprüchen beschrieben.

Diese Aufgabe löst die Erfindung dadurch, dass die Gewebemarkierung einen Transponder umfasst, wobei der Transponder durch elektromagnetische Strahlung aktivierbar ist, und zwar in der Weise, dass der Transponder ein Lokalisierungssignal, anhand dessen die Position der Gewebemarkierung im Körpergewebe feststellbar ist, als elektromagnetische Strahlung aussendet.

Die Grundidee der Erfindung ist die Verwendung eines Transponders an sich bekannter Art als Gewebemarkierung zur Markierung einer Läsion im Körpergewebe.

Besonders gut eignet sich als Gewebemarkierung ein RFID-Tag. RFID ist bekanntlich ein Verfahren zur berührungslosen Identifizierung und Lokalisierung. Ein RFID-System besteht aus einem Transponder, der sich an dem zu identifizierenden Objekt befindet und dieses kennzeichnet, und einem Lesegerät zum Auslesen der Transponder-Kennung. Ein RFID-Transponder (auch als RFID-Tag bezeichnet) umfasst üblicherweise eine Antenne sowie einen integrierten elektronischen Schaltkreis mit einem analogen und einem digitalen Teil. Der analoge Teil (Transceiver) dient zum Empfangen und Senden von elektromagnetischer Strahlung. Der digitale Schaltkreis weist einen Datenspeicher auf, in welchem Identifizierungsdaten des Transponders speicherbar sind. Bei komplexeren RFID-Transpondern hat der digitale Teil des Schaltkreises eine Von-Neumann-Architektur. Das von dem Lesegerät erzeugte hochfrequente elektromagnetische Feld wird über die Antenne des RFID-Transponders empfangen. In der Antenne entsteht, sobald sie sich in dem elektromagnetischen Feld des Lesegerätes befindet, ein Induktionsstrom, durch welchen der Transponder aktiviert wird. Der so aktivierte Transponder empfängt über das elektromagnetische Feld Befehle vom Lesegerät. Der Transponder erzeugt ein Antwortsignal, welches die vom Lesegerät abgefragten Daten enthält. Gemäß der Erfindung ist das Antwortsignal das Lokalisierungssignal, anhand dessen die Position der Gewebemarkierung im Körpergewebe festgestellt wird.

Die erfindungsgemäße Verwendung eines RFID-Transponders als Gewebemarkierung hat somit den Vorteil, dass die Gewebemarkierung vollständig in das Körpergewebe implantiert werden kann, ohne dass, wie bei den bekannten Drahtführungen, ein die Haut durchdringender Führungsdraht dauerhaft im Körper verbleibt.

RFID-Tags eignen sich besonders gut als Gewebemarkierung, da diese eine sehr geringe Baugröße haben. Mit heutiger Technik ist es möglich, miniaturisierte RFID-Transponder herzustellen, die nur staubkorngroß sind. Bekannt sind RFID-Transponder mit einer Größe von 0,05 x 0,05 mm. Derartige Transponder arbeiten bei sehr hohen Frequenzen im Bereich von 1 Gigahertz und darüber. Ein derartig miniaturisierter RFID-Transponder kann problemlos im Körpergewebe verbleiben. Eine operative Entfernung nach Abschluss einer Therapie ist nicht erforderlich. Auch können die sehr kleinen RFID-Transponder sehr einfach an die gewünschte Stelle im Körpergewebe implantiert werden. Im einfachsten Fall erfolgt dies durch eine Hohlnadel geringen Durchmessers unter Überwachung durch ein bildgebendes Gerät.

Der Transponder der erfindungsgemäßen Gewebemarkierung ist zweckmäßigerweise als passiver Transponder ausgebildet, wobei die Stromversorgung des Schaltkreises durch den beim Empfang von elektromagnetischer Strahlung in der Antenne erzeugten Induktionsstrom erfolgt. Von Vorteil ist die geringe Baugröße von passiven Transpondern, da diese ohne eigene aktive Energieversorgung, beispielsweise in Form einer Batterie, auskommen. Die Energie, die der Transponder zum Aussenden des Lokalisierungssignals benötigt, wird durch die elektromagnetische Strahlung, durch welche die Aktivierung des Transponders erfolgt, zur Verfügung gestellt. Zweckmäßigerweise weist der Transponder zur Stromversorgung seines integrierten Schaltkreises einen Kondensator auf, der durch den in der Antenne erzeugten Induktionsstrom geladen wird. Da die dauerhafte Energieversorgung durch den Kondensator gewährleistet ist, kann die Antenne des Transponders sehr klein dimensioniert werden, was wiederum von Vorteil für die Verwendung des Transponders als Gewebemarkierung ist.

Gemäß einer sinnvollen Weiterbildung der erfindungsgemäßen Gewebemarkierung ist der Transponder mit wenigstens einem Sensorelement verbunden, wobei der Transponder ein Sensorsignal des Sensorelements als elektromagnetische Strahlung aussendet. Demgemäß wird der Transponder nicht nur zur Markierung einer Läsion im Körpergewebe, sondern auch zur Übertragung von Sensorsignalen, die an der entsprechenden Läsionsstelle aufgenommen werden, benutzt. Der Transponder ist mit entsprechenden Sensorelementen, beispielsweise einem Temperatursensor, einem Drucksensor oder einem pH-Sensor, verbunden. Der Transponder überträgt das Sensorsignal drahtlos als Analog- oder Digitalsignal.

Erfindungsgemäß ist die Gewebemarkierung biodegradierbar.

Wenn die Gewebemarkierung biodegradierbar ist, kann eine operative Entfernung nach Abschluss einer Therapie, für welche die Gewebemarkierung benötigt wird, gänzlich entfallen, und zwar unabhängig von der Größe der Gewebemarkierung. Auch ein größer dimensionierter Transponder kann verwendet werden, wenn die Gewebemarkierung biodegradierbar ist. Es ist möglich, sämtliche Komponenten der Gewebemarkierung einschließlich des Transponders aus biodegradierbaren Materialien herzustellen und die Oberflächen sämtlicher Teile der Gewebemarkierung so auszugestalten, dass die biologische Abbaubarkeit gewährleistet ist. Der integrierte Schaltkreis des Transponders besteht im Wesentlichen aus Silizium, das sowohl biodegradierbar als auch biokompatibel ist. Für die Antenne und gegebenenfalls für weitere Komponenten der Gewebemarkierung können ohne Weiteres bekannte biodegradierbare und biokompatible Materialien gewählt werden. Besonders zweckmäßig ist es, den Transponder der erfindungsgemäßen Gewebemarkierung in eine biodegradierbare Umhüllung einzubetten, wobei die biologische Abbauzeit durch die Dicke und/oder durch die Zusammensetzung der Umhüllung vorgebbar sind. Auf diese Weise lässt sich die biologische Abbauzeit an die Dauer der Therapie, für welche die Gewebemarkierung benutzt wird, anpassen. Verschiedene polymere, keramische und metallische Materialien, die als biodegradierbar und biokompatibel bekannt sind, eignen sich als Umhüllung für die erfindungsgemäße Gewebemarkierung.

Mit heutiger Technik sind RFID-Tags im Druckverfahren herstellbar. Auf diese Weise gedruckte RFID-Tags eignen sich gut als Gewebemarkierungen gemäß der Erfindung. Es ist beispielsweise denkbar, ein RFID-Tag als Gewebemarkierung auf ein Substrat aus biodegradierbarem Material zu drucken.

Wie oben erwähnt, ist es für die präzise Durchführung eines operativen Eingriffs wichtig, den Ort der Läsion, das heißt die Position des Zentrums der Läsion, sowie auch die Ränder der Läsion möglichst genau lokalisieren zu können. Hierzu können mit Vorteil eine Mehrzahl von erfindungsgemäßen Gewebemarkierungen benutzt werden. Beispielsweise kann eine einzelne Gewebemarkierung benutzt werden, um das Zentrum einer Läsion zu markieren, während weitere Gewebemarkierungen am Rand der Läsion platziert werden, um die genaue Begrenzung der Läsion für den operierenden Arzt erkennbar zu machen. Die einzelnen RFID-Tags der Gewebemarkierung können in ihrem elektronischen Datenspeicher medizinische Daten bezüglich der zu markierenden Läsion speichern, so dass der Arzt bei der Abfrage der jeweiligen RFID-Tags weitere Informationen über die Läsion erhält, die für die Durchführung des Eingriffs wichtig sind. So kann in dem Datenspeicher eines RFID-Tags, das als Gewebemarkierung zur Markierung des Zentrums einer Läsion verwendet wird, eine Information über die Größe der Läsion enthalten sein. Auf diese Weise erhält der Arzt Informationen über die zu entfernende Gewebemenge, um zu gewährleisten, dass die gesamte Läsion entfernt wird. Da die einzelnen RFID-Tags aufgrund ihrer individuellen Identifikationsdaten unterscheidbar sind, können diese genutzt werden, um gleichsam "Wegpunkte" für einen durchzuführenden operativen Eingriff zu markieren. Diese "Wegpunkte" kann der Arzt in einer vorher festgelegten Reihenfolge bei der Durchführung eines operativen Eingriffs nacheinander ansteuern.

Ein System zur Bestimmung der Position einer erfindungsgemäßen Gewebemarkierung im Körpergewebe umfasst eine Sendeeinheit zum Aussenden von elektromagnetischer Strahlung zur Aktivierung des Transponders der Gewebemarkierung, eine Empfangseinheit zum Empfangen des von dem Transponder ausgesendeten Lokalisierungssignals sowie eine Auswertungseinheit zur Auswertung des Lokalisierungssignals. Die Sendeeinheit, die Empfangseinheit und die Auswertungseinheit bilden zusammen ein Lesegerät, wie es zum Auslesen von RFID-Tags im Prinzip üblich ist, wobei die Auswertungseinheit um Funktionen zur Bestimmung der Position der Gewebemarkierung anhand des Lokalisierungssignals erweitert ist. Denkbar ist es, mehrere Empfangseinheiten zu verwenden, welche das von dem Transponder ausgesendete Lokalisierungssignal empfangen. Aus der Feldstärke des Lokalisierungssignals am Ort der jeweiligen Empfangseinheit kann auf den Abstand des Transponders von der Empfangseinheit zurückgeschlossen werden. Wenn die Abstände des Transponders von den verschiedenen Empfangseinheiten, die sich an definierten Positionen im Raum befinden, bekannt sind, kann daraus mittels der Auswertungseinheit wiederum die genaue Position des Transponders und damit der Gewebemarkierung im Körpergewebe berechnet werden.

Problematisch ist, dass die Feldstärke des Lokalisierungssignals durch das Körpergewebe abgeschwächt wird. Das Körpergewebe absorbiert aufgrund seiner elektrischen Eigenschaften die von dem Transponder emittierte elektromagnetische Strahlung teilweise. Aus diesem Grund ist eine Positionsbestimmung anhand der Feldstärke des Lokalisierungssignals unter Umständen nicht immer mit ausreichender Genauigkeit möglich. Zur Lösung dieses Problems ist die Auswertungseinheit zur Bestimmung der Position der Gewebemarkierung anhand der Phasenlage der elektromagnetischen Strahlung des Lokalisierungssignals am Ort der jeweiligen Empfangseinheit eingerichtet.

Bei geeigneter Wahl der Frequenz des Lokalisierungssignals ist der Einfluss der elektrischen Eigenschaften des Körpergewebes auf die Phase des Lokalisierungssignals gering. Der Transponder sollte dabei so eingerichtet sein, dass dieser das Lokalisierungssignals kohärent, das heißt mit definierter und konstanter Phasenlage, emittiert.

Wenn die Positionsbestimmung, wie zuvor beschrieben, anhand der Phasenlage der elektromagnetischen Strahlung des Lokalisierungssignals erfolgt, ist zu beachten, dass eine eindeutige Zuordnung eines Phasenwertes zu einer Position im Raum nur innerhalb einer Distanz möglich ist, die kleiner ist als die Wellenlänge des Lokalisierungssignals. Bei größeren Distanzen ist es zusätzlich erforderlich, die Zahl der Nulldurchgänge der elektromagnetischen Wellen zwischen dem Transponder und der jeweiligen Empfangseinheit zu bestimmen.

Zur Erzielung einer möglichst hohen Genauigkeit bei der Positionsbestimmung kann es sinnvoll sein, den Transponder und die zugehörigen Empfangseinheiten so auszugestalten, dass diese bei zwei oder mehr verschiedenen Frequenzen arbeiten. Dadurch lässt sich ein abgestuftes Lokalisierungsverfahren zur sukzessiven Erhöhung der Genauigkeit bei der Positionsbestimmung realisieren. Durch die Erzeugung des Lokalisierungssignals bei niedrigen Frequenzen und entsprechend großen Wellenlängen kann zunächst eine grobe aber eindeutige Bestimmung der Position erfolgen. Zur Steigerung der Genauigkeit wird dann zu einer höheren Frequenz übergegangen, oder es wird die Frequenz des Lokalisierungssignals sukzessive weiter gesteigert. Bei höheren Frequenzen sind die Anforderungen an die Auflösung bei der Bestimmung der Phasenlage zur Erzielung einer bestimmten räumlichen Auflösung geringer. Bei der sukzessiven Steigerung der Frequenz kann die Zahl der Nulldurchgänge zur Bestimmung des genauen Abstands zwischen Transponder und Empfangseinheit ermittelt werden. Zur möglichst genauen Positionsbestimmung ist eine Frequenzänderung in beiden Richtungen, das heißt von niedrigen zu hohen Frequenzen oder auch von hohen zu niedrigen Frequenzen, denkbar. In Abhängigkeit von den Frequenzbereichen, die zur Positionsbestimmung abgedeckt werden müssen, kann es erforderlich sein, zwei oder mehr Antennen vorzusehen, mit denen der Schaltkreis des Transponders verbunden ist, wobei jede der Antennen jeweils einem bestimmten Frequenzbereich zugeordnet ist. Ebenso denkbar ist es, Gewebemarkierungen zu verwenden, die jeweils mehrere separate Transponder umfassen, die bei verschiedenen Frequenzen arbeiten.

Gemäß einer zweckmäßigen Weiterbildung des Systems ist eine Zielvorrichtung zur Bestimmung der Position eines medizinischen Instrumentes relativ zur Position der Gewebemarkierung vorgesehen. Die Zielvorrichtung bestimmt die Position und/oder die Ausrichtung eines für einen operativen Eingriff verwendeten medizinischen Instrumentes relativ zu der erfindungsgemäßen Gewebemarkierung. Auf diese Weise kann die Zielvorrichtung einem Arzt anzeigen, in welche Richtung dieser das medizinische Instrument führen muss, um mit dem Instrument (oder dessen Spitze) zu der Position der Gewebemarkierung und damit zu der Läsionsstelle zu gelangen. Gleichzeitig kann die Zielvorrichtung dem Arzt den Abstand des Instrumentes von der Gewebemarkierung anzeigen, so dass der Arzt eine Information über die Wegstrecke erhält, um die das Instrument vorgeschoben werden muss, um die Läsionsstelle zu erreichen. Die Anzeige der Zielvorrichtung kann beispielsweise eine Pfeildarstellung umfassen, wobei dem Arzt durch die Pfeile angezeigt wird, in welche Richtung er das Instrument bewegen muss. Ebenso denkbar ist eine Anzeige durch ein akustisches Signal oder einen Vibrationssignalgeber. Die Zielvorrichtung kann direkt an dem medizinischen Instrument angebracht oder anbringbar sein. Ebenso gut kann die Zielvorrichtung von dem medizinischen Instrument separat sein. Zur Bestimmung der Position und/oder der Orientierung des medizinisches Instrumentes relativ zur Position der Gewebemarkierung kann das medizinische Instrument wiederum mit einem oder mehreren RFID-Tags versehen sein, deren Position in der oben beschriebenen Art und Weise bestimmt wird. Denkbar ist es, ein RFID-Tag an dem medizinischen Instrument durch ein Druckverfahren an sich bekannter Art anzubringen. Alternativ können die Sende- und/oder Empfangseinheit des erfindungsgemäßen Systems mit dem medizinischen Instrument verbunden sein, um auf diese Weise die Relativposition von Instrument und Gewebemarkierung zu erfassen. Die Sende- und/oder die Empfangseinheit des Systems können sinnvollerweise eine Richtcharakteristik aufweisen, um die Richtung des von der Gewebemarkierung abgestrahlten Lokalisierungssignals in Bezug auf die Ausrichtung des medizinischen Instruments zu erfassen.

Bei Verwendung einer erfindungsgemäßen Gewebemarkierung kann sich das Problem ergeben, dass die Markierung im Körpergewebe "wandert", so dass der Ort der Läsion nicht mehr mit dem Ort der Gewebemarkierung übereinstimmt. Um dies überprüfen und ausschließen zu können, ist es sinnvoll, zwei oder mehr Gewebemarkierungen in einer definierten geometrischen Konfiguration zu implantieren. Stellt man bei der Überprüfung der Positionen der einzelnen Gewebemarkierungen fest, dass sich deren relative Anordnung im Körper gegenüber der ursprünglichen Konfiguration geändert hat, deutet dies auf ein "Wandern" zumindest einer der Markierungen hin. In diesem Fall ist die Markierung als nicht mehr brauchbar zu qualifizieren. Es ist als extrem unwahrscheinlich anzusehen, dass mehrere Gewebemarkierungen in identischer Weise "wandern" und dabei ihre relative geometrische Konfiguration beibehalten.

Die erfindungsgemäße Gewebemarkierung eignet sich zur Fokusmarkierung in der Radiotherapie. Die markierte Läsion kann nach Bestimmung der Position der Gewebemarkierung im Körpergewebe automatisch in den Strahlengang bzw. in den Fokus der radiotherapeutischen Strahlungsquelle gebracht werden. Ebenso kann die Strahlungsquelle bzw. deren Kollimationsoptik automatisch auf die Läsionsstelle ausgerichtet werden. Sehr gut ist auch die automatische Einstellung des Kollimators der Strahlungsquelle auf die Zielkontur entsprechend den Abgrenzungen der (vorzugsweise mit mehreren erfindungsgemäßen Gewebemarkierungen markierten) Läsion möglich.

Ausführungsbeispiele der Erfindung werden im Folgenden anhand der Figuren näher erläutert.

Es zeigen:
- Figur 1: schematische Darstellung einer erfindungsgemäßen Gewebemarkierung;
- Figur 2: erfindungsgemäßes System zur Bestimmung der Position einer Gewebemarkierung;
- Figur 3: medizinisches Instrument mit erfindungsgemäßer Zielvorrichtung.

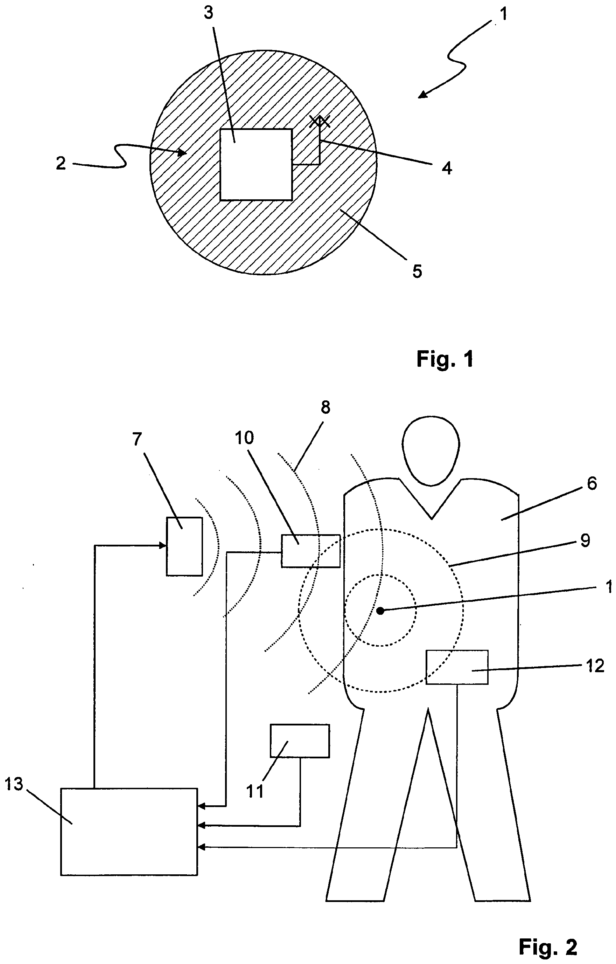

Die in der Figur 1 gezeigte Gewebemarkierung ist insgesamt mit der Bezugsziffer 1 bezeichnet. Diese umfasst einen Transponder 2, der aus einem integrierten elektronischen Schaltkreis 3 und einer Antenne 4 besteht. Die Gewebemarkierung 1 ist biodegradierbar ausgestaltet. Um die biologische Abbauzeit gezielt vorgeben zu können, weist die Gewebemarkierung 1 eine den Transponder 2 einschließende biodegradierbare Umhüllung 5 auf. Die biologische Abbauzeit ist durch die Dicke, die Zusammensetzung und durch die Oberflächenstruktur der Umhüllung 5 vorgegeben. Durch geeignete Oberflächenstrukturierung der Umhüllung 5 kann des Weiteren ein "Wandern" der Gewebemarkierung im Körpergewebe verhindert werden. Eine raue bzw. poröse Oberflächenstrukturierung ist für die Biodegradierbarkeit förderlich und bewirkt gleichzeitig ein Einwachsen von Gewebe in die Umhüllung, so dass die Gewebemarkierung 1 an der zu markierenden Läsionsstelle sicher fixiert ist.

Das in der Figur 2 dargestellte System dient zur Bestimmung der Position einer Gewebemarkierung 1 im Körpergewebe 6 eines Patienten. Das System umfasst eine Sendeeinheit 7, die elektromagnetische Strahlung 8 aussendet. Die Strahlung 8 wird von dem Transponder der Gewebemarkierung 1 empfangen. Der Transponder wird durch die empfangene Strahlung 8 angeregt, so dass dieser seinerseits ein Lokalisierungssignal als hochfrequente elektromagnetische Strahlung 9 aussendet. Das von dem Transponder der Gewebemarkierung 1 abgestrahlte Lokalisierungssignal 9 wird von drei an definierten Positionen im Raum befindlichen Empfangseinheiten 10, 11 und 12 empfangen. Die Empfangseinheiten 10, 11 und 12 sind mit einer Auswertungseinheit 13 verbunden, welche anhand der Intensität und anhand der Phasenlage der elektromagnetischen Strahlung des Lokalisierungssignals 9 am jeweiligen Ort der Empfangseinheiten 10, 11 und 12 die Position der Gewebemarkierung 1 berechnet.

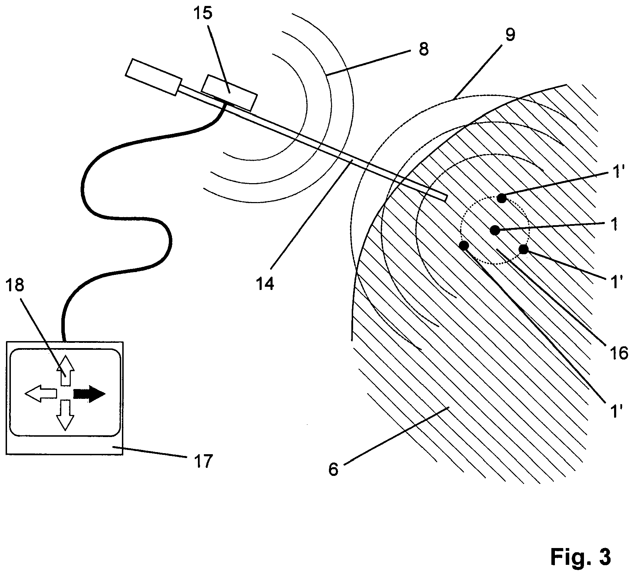

Die Figur 3 zeigt ein medizinisches Instrument 14 mit einer Zielvorrichtung 15. Die Zielvorrichtung 15 dient zur Bestimmung des Position des medizinischen Instrumentes 14 relativ zur Position von Gewebemarkierungen 1 bzw. 1' die sich im Körpergewebe 6 befinden. Die Gewebemarkierung 1 dient zur Markierung des Zentrums einer Läsion 16. Die Gewebemarkierungen 1' sind entlang des Randes der Läsion 16 angeordnet, um die Größe der Läsion 16 erkennbar zu machen. Die Zielvorrichtung 15 umfasst die Sendeeinheit 7 sowie eine Empfangseinheit 10 (siehe Figur 2). Die Zielvorrichtung 15 sendet elektromagnetische Strahlung 8 aus, durch welche die Transponder der Gewebemarkierungen 1 bzw. 1' aktiviert und zum Abstrahlen eines Lokalisierungssignals 9 angeregt werden. Anhand des über die Zielvorrichtung 15 empfangenen Lokalisierungssignals 9 bestimmt diese die relative Position und/oder Orientierung des Instrumentes 14 relativ zu den Gewebemarkierungen 1 bzw. 1'. Die einzelnen Gewebemarkierungen 1 bzw. 1' sind anhand der Identifizierungsdaten der einzelnen Transponder unterscheidbar. Bei der Durchführung einer Operation kann der Arzt eine bestimmte Gewebemarkierung 1 bzw. 1' auswählen, die er mit dem Instrument 14 ansteuern möchte. Die Zielvorrichtung 15 ist mit einer Anzeige 17 verbunden, welche dem Arzt die Führungsrichtung bzw. die Orientierung des medizinischen Instrumentes 14 anzeigt, so dass dieses zu der Position der ausgewählten Gewebemarkierung 1 bzw. 1' geführt wird. Hierzu zeigt die Anzeige 17 Pfeile 18 an, die dem Arzt einen eindeutigen Hinweis geben, um das Instrument 14 korrekt auszurichten.

## Patentansprüche

1. System zur Bestimmung der Position einer zur Markierung einer Läsion (16) im Körpergewebe dienenden Gewebemarkierung (1, 1') im Körpergewebe (6), wobei die Gewebemarkierung (1, 1') einen Transponder (2) umfasst, wobei der Transponder (2) durch elektromagnetische Strahlung (8) aktivierbar ist, und zwar in der Weise, dass der Transponder (2) ein Lokalisierungssignal (9), anhand dessen die Position der Gewebemarkierung (1) im Körpergewebe (6) feststellbar ist, als elektromagnetische Strahlung aussendet, wobei das System eine Sendeeinheit (7) zum Aussenden von elektromagnetischer Strahlung (8) zur Aktivierung des Transponders (2) der Gewebemarkierung (1, 1'), eine Empfangseinheit (10, 11, 12) zum Empfangen des von dem Transponder ausgesendeten Lokalisierungssignals (9) und eine Auswertungseinheit (13) zur Auswertung des Lokalisierungssignals (9) aufweist, **dadurch gekennzeichnet, dass** die Auswertungseinheit (13) zur Bestimmung der Position der Gewebemarkierung (1, 1') anhand der Phasenlage der elektromagnetischen Strahlung des Lokalisierungssignals (9) am Ort der Empfangseinheit (10, 11, 12) eingerichtet ist und dass die Gewebemarkierung (1) einschließlich des Transponders biodegradierbar ist.

2. System nach Anspruch 1, **gekennzeichnet durch** eine Zielvorrichtung (15) zur Bestimmung der Position und/oder Orientierung eines medizinischen Instrumentes (14) relativ zur Position der Gewebemarkierung (1, 1').

3. System nach Anspruch 2, **dadurch gekennzeichnet, dass** die Zielvorrichtung (15) eine Anzeige (17) aufweist, welche einer das medizinische Instrument (14) führenden Person die Führungsrichtung und/oder die Orientierung des medizinischen Instrumentes (14) anzeigt, so dass dieses zu der Position der Gewebemarkierung (1, 1') geführt wird.

4. System nach Anspruch 3, **dadurch gekennzeichnet, dass** die Zielvorrichtung (15) an dem medizinischen Instrument (14) angebracht oder anbringbar ist.

5. System nach einem der Ansprüche 1 bis 4, **dadurch gekennzeichnet, dass** die Sende- und/oder die Empfangseinheit (7, 10, 11, 12) eine Richtcharakteristik aufweisen.

6. System nach einem der Ansprüche 1 bis 5, **dadurch gekennzeichnet, dass** mittels der Auswertungseinheit (13) die Gewebemarkierung (1, 1') anhand eines von dem Transponder (2) der Gewebemarkierung (1) ausgesendeten und über die Empfangseinheit (10, 11, 12) empfangenen Identifizierungssignals identifizierbar ist.

7. System nach einem der Ansprüche 1 bis 6, **dadurch gekennzeichnet, dass** der Transponder (2) einen integrierten elektronischen Schaltkreis (3) und eine damit verbundene Antenne (4) zum Empfang und zum Aussenden von elektromagnetischer Strahlung aufweist.

8. System nach Anspruch 7, **dadurch gekennzeichnet, dass** der Transponder (2) als passiver Transponder ausgebildet ist, wobei die Stromversorgung des Schaltkreises (3) durch den beim Empfang von elektromagnetischer Strahlung in der Antenne (4) erzeugten Induktionsstrom erfolgt.

9. System nach einem der Ansprüche 1 bis 8, **dadurch gekennzeichnet, dass** die Gewebemarkierung (1, 1') ein mit dem Transponder (2) verbundenes Sensorelement aufweist, wobei der Transponder (2) ein Sensorsignal des Sensorelementes als elektromagnetische Strahlung aussendet.

10. System nach Anspruch 1, **dadurch gekennzeichnet, dass** die Gewebemarkierung (1, 1') eine den Transponder (2) einschließende biodegradierbare Umhüllung (5) aufweist, wobei die biologische Abbauzeit durch die Dicke und/oder durch die Zusammensetzung der Umhüllung (5) vorgebbar sind.

11. System nach einem der Ansprüche 1 bis 10, **dadurch gekennzeichnet, dass** der Transponder (2) ein RFID-Tag ist.

12. System nach Anspruch 11, **dadurch gekennzeichnet, dass** in einem elektronischen Datenspeicher des RFID-Tags medizinische Daten bezüglich der zu markierenden Läsion gespeichert oder speicherbar sind.

13. Verfahren zur Bestimmung der Position einer Gewebemarkierung (1, 1') im Körpergewebe (6) mit einem System nach einem der Ansprüche 1 bis 12 wobei, mittels einer Sendeeinheit (7) ein elektromagnetisches Signal (8) ausgesendet wird, das von einem Transponder (2) der Gewebemarkierung (1, 1') empfangen wird, woraufhin der Transponder (2) ein Lokalisierungssignal (9) aussendet, das über eine Empfangseinheit (10, 11, 12) empfangen wird, wobei mittels einer Auswertungseinheit (13), die mit der Empfangseinheit (10, 11, 12) verbunden ist, aus dem Lokalisierungssignal (9) die Position der Gewebemarkierung (1, 1') bestimmt wird.

14. Verfahren nach Anspruch 13, **dadurch gekennzeichnet, dass** mittels einer Zielvorrichtung (15) die Position und/oder die Orientierung eines medizinischen Instrumentes (14) relativ zur Position der Gewebemarkierung (1, 1') bestimmt wird, wobei einer das medizinische Instrument (14) führenden Person die Führungsrichtung und/oder die Orientierung des medizinischen Instrumentes (14) angezeigt wird.

## Claims

1. System for determining the position of a tissue marker (1, 1'), serving to mark a lesion (16) in the body tissue, in the body tissue (6), wherein the tissue marker (1, 1') comprises a transponder (2), wherein the transponder (2) is activatable by electromagnetic radiation (8), to be precise in such a way that the transponder (2) emits a localization signal (9), on the basis of which the position of the tissue marker (1) in the body tissue (6) is determinable, as electromagnetic radiation, wherein the system comprises a transmission unit (7) for emitting electromagnetic radiation (8) for activating the transponder (2) of the tissue marker (1, 1'), a reception unit (10, 11, 12) for receiving the localization signal (9) emitted by the transponder and an evaluation unit (13) for evaluating the localization signal (9),
**characterized**
**in that** the evaluation unit (13) is configured to determine the position of the tissue marker (1, 1') on the basis of the phase of the electromagnetic radiation of the localization signal (9) at the location of the reception unit (10, 11, 12) and in that the tissue marker (1), including the transponder, is biodegradable.

2. System according to Claim 1, **characterized by** a targeting device (15) for determining the position and/or orientation of a medical instrument (14) relative to the position of the tissue marker (1, 1').

3. System according to Claim 2, **characterized in that** the targeting device (15) has a display (17) which indicates the guiding direction and/or the orientation of the medical instrument (14) to a person guiding the medical instrument (14) such that said medical instrument is guided to the position of the tissue marker (1, 1').

4. System according to Claim 3, **characterized in that** the targeting device (15) is attached or attachable to the medical instrument (14).

5. System according to one of Claims 1 to 4,
**characterized in that** the transmission unit (7) and/or the reception unit (10, 11, 12) has/have a directionality.

6. System according to one of Claims 1 to 5,
**characterized in that** the tissue marker (1, 1') is identifiable by means of the evaluation unit (13) on the basis of an identification signal which is emitted by the transponder (2) of the tissue marker (1) and received by the reception unit (10, 11, 12).

7. System according to one of Claims 1 to 6,
**characterized in that** the transponder (2) has an integrated electronic circuit (3) and an antenna (4) connected therewith for receiving and emitting electromagnetic radiation.

8. System according to Claim 7, **characterized in that** the transponder (2) is embodied as a passive transponder, wherein the power supply of the circuit (3) is effected by the induction current generated when receiving electromagnetic radiation in the antenna (4).

9. System according to one of Claims 1 to 8,
**characterized in that** the tissue marker (1, 1') has a sensor element connected to the transponder (2), the transponder (2) emitting a sensor signal of the sensor element as electromagnetic radiation.

10. System according to Claim 1, **characterized in that** the tissue marker (1, 1') has a biodegradable sleeve (5) enclosing the transponder (2), the biological decomposition time being predeterminable by the thickness and/or the composition of the sleeve (5).

11. System according to one of Claims 1 to 10,
**characterized in that** the transponder (2) is an RFID tag.

12. System according to Claim 11, **characterized in that** medical data in respect of the lesion to be marked are stored or storable in an electronic data storage of the RFID tag.

13. Method for determining the position of a tissue marker (1, 1') in the body tissue (6) using a system according to one of Claims 1 to 12, wherein an electromagnetic signal (8) which is received by a transponder (2) of the tissue marker (1, 1') is emitted by means of a transmission unit (7), whereupon the transponder (2) emits a localization signal (9) which is received by way of a reception unit (10, 11, 12), the position of the tissue marker (1, 1') being determined from the localization signal (9) by way of an evaluation unit (13) connected to the reception unit (10, 11, 12).

14. Method according to Claim 13, **characterized in that** the position and/or the orientation of a medical instrument (14) relative to the position of the tissue marker (1, 1') is determined by means of a targeting device (15), wherein the guiding direction and/or the orientation of the medical instrument (14) is indicated to a person guiding the medical instrument (14).

## Revendications

1. Système de détermination de la position d'un marquage tissulaire (1, 1') dans le tissu organique (6) servant au marquage d'une lésion (16) dans le tissu organique, le marquage tissulaire (1, 1') comprenant un transpondeur (2), le transpondeur (2) pouvant être activé par un rayonnement électromagnétique (8), et ce de sorte que le transpondeur (2) émet en tant que rayonnement électromagnétique un signal de localisation (9) au moyen duquel peut être définie la position du marquage tissulaire (1) dans le tissu organique (6), le système possédant une unité d'émission (7) destinée à émettre un rayonnement électromagnétique (8) destiné à l'activation du transpondeur (2) du marquage tissulaire (1, 1'), une unité de réception (10, 11, 12) destinée à recevoir le signal de localisation (9) émis par le transpondeur et une unité d'interprétation (13) destinée à interpréter le signal de localisation (9), **caractérisé en ce que** l'unité d'interprétation (13) est conçue pour déterminer la position du marquage tissulaire (1, 1') au moyen de la position de phase du rayonnement électromagnétique du signal de localisation (9) à l'endroit de l'unité de réception (10, 11, 12) et **en ce que** le marquage tissulaire (1), y compris le transpondeur, sont biodégradables.

2. Système selon la revendication 1, **caractérisé par** un dispositif de visée (15) destiné à déterminer la position et/ou l'orientation d'un instrument médical (14) par rapport à la position du marquage tissulaire (1, 1').

3. Système selon la revendication 2, **caractérisé en ce que** le dispositif de visée (15) possède un indicateur (17) qui indique à une personne guidant l'instrument médical (14) le sens de guidage et/ou l'orientation de l'instrument médical (14), de sorte que celui-ci est guidé vers la position du marquage tissulaire (1, 1').

4. Système selon la revendication 3, **caractérisé en ce que** le dispositif de visée (15) est monté ou peut être monté sur l'instrument médical (14).

5. Système selon l'une des revendications 1 à 4, **caractérisé en ce que** l'unité d'émission et/ou de réception (7, 10, 11, 12) possèdent une caractéristique d'orientation.

6. Système selon l'une des revendications 1 à 5, **caractérisé en ce que** le marquage tissulaire (1, 1') peut être identifié au moyen de l'unité d'interprétation (13) à l'aide d'un signal d'identification émis par le transpondeur (2) du marquage tissulaire (1) et reçu par le biais de l'unité de réception (10, 11, 12).

7. Système selon l'une des revendications 1 à 6, **caractérisé en ce que** le transpondeur (2) possède un circuit électronique intégré (3) et une antenne (4) reliée à celui-ci servant à la réception et à l'émission d'un rayonnement électromagnétique.

8. Système selon la revendication 7, **caractérisé en ce que** le transpondeur (2) est réalisé sous la forme d'un transpondeur passif, l'alimentation électrique du circuit (3) s'effectuant par le biais du courant d'induction généré dans l'antenne (4) lors de la réception du rayonnement électromagnétique.

9. Système selon l'une des revendications 1 à 8, **caractérisé en ce que** le marquage tissulaire (1, 1') possède un élément de détection relié au transpondeur (2), le transpondeur (2) émettent un signal de détection de l'élément de détection sous la forme d'un rayonnement électromagnétique.

10. Système selon la revendication 1, **caractérisé en ce que** le marquage tissulaire (1, 1') possède un transpondeur (2) incluant un enrobage (5) biodégradable, le temps de biodégradation pouvant être prédéfini par l'épaisseur et/ou par la composition de l'enrobage (5).

11. Système selon l'une des revendications 1 à 10, **caractérisé en ce que** le transpondeur (2) est une balise RFID.

12. Système selon la revendication 11, **caractérisé en ce que** des données médicales concernant la lésion marquée sont enregistrées ou peuvent être enregistrées dans une mémoire de données électronique de la balise RFID.

13. Procédé de détermination de la position d'un marquage tissulaire (1, 1') dans un tissu organique (6) avec un système selon l'une des revendications 1 à 12, un signal électromagnétique (8) étant émis au moyen d'une unité d'émission (7), lequel est reçu par un transpondeur (2) du marquage tissulaire (1, 1'), suite à quoi le transpondeur (2) émet un signal de localisation (9) qui est reçu par le biais d'une unité de réception (10, 11, 12), la position du marquage tissulaire (1, 1') étant déterminée à partir du signal de localisation (9) au moyen d'une unité d'interprétation (13), laquelle est reliée à l'unité de réception (10, 11, 12).

14. Procédé selon la revendication 13, **caractérisé en ce que** la position et/ou l'orientation d'un instrument médical (14) par rapport à la position du marquage tissulaire (1, 1') sont déterminées au moyen d'un dispositif de visée (15), le sens de guidage et/ou l'orientation de l'instrument médical (14) étant indiqués à une personne guidant l'instrument médical (14).
